# EUROPEAN PATENT APPLICATION

(11) **EP 4 249 459 A1**
(43) Date of publication of application: **27.09.2023**
(21) Application number: 21910815.6
(22) Date of filing: 21.12.2021
(51) Int. Cl.: C07C 21/18, C07C 17/358, C07B 61/00, B01J 27/125

(54) **METHOD FOR PRODUCING UNSATURATED CHLOROFLUOROCARBON, AND COMPOSITION**

(30) Priority: 22.12.2020 JP 2020212438
(71) Applicant: Central Glass Co., Ltd., Yamaguchi 755-0001 (JP)
(72) Inventor: NISHINAKA Naoki, Kawagoe-shi, Saitama 350-1159 (JP); IMURA Hideaki, Kawagoe-shi, Saitama 350-1159 (JP); OKAMOTO Masamune, Kawagoe-shi, Saitama 350-1159 (JP)
(74) Representative: Manitz Finsterwald Patent- und Rechtsanwaltspartnerschaft mbB
(86) International application number: PCT/JP2021/047427
(87) International publication number: WO 2022/138675

(57) **Abstract**

To provide a method and a composition for producing a geometric isomer (isomer 2) represented by a certain formula (1) by geometrically isomerizing a corresponding geometric isomer (isomer 1) represented by the certain formula (1), the method including, contacting the geometric isomer (isomer 1) represented by the certain formula (1) with a compound (A) which is at least one of a dichlorotrifluoropropane and hydrogen chloride in a gas phase.

## Description

### TECHNICAL FIELD

The present disclosure relates to a method for producing a unsaturated chlorofluorocarbon, and a composition, and relates to a method for producing a geometric isomer (isomer 2) by geometrically isomerizing a 1-chloro-3,3,3-trifluoropropene or 1-chloro-2,3,3-trifluoropropene geometric isomer (isomer 1) corresponding thereto, and a composition.

### BACKGROUND ART

Unsaturated chlorofluorocarbons such as 1-chloro-3,3,3-trifluoropropene and 1-chloro-2,3,3-trifluoropropene have a low ozone depletion potential (ODP) and global warming potential (GWP), and are thus expected to be one of compounds that can be used for cleaning agents, foaming agents, refrigerants, or the like. For example, Patent Literature 1 discloses a method for isomerizing a cis isomer of 1-chloro-3,3,3-trifluoropropene to a trans isomer, and a method for isomerizing a trans isomer of 1-chloro-3,3,3-trifluoropropene to a cis isomer.

### CITATION LIST

### PATENT LITERATURE

Patent Literature 1: JP6156374B

### SUMMARY OF INVENTION

### TECHNICAL PROBLEM

An object of embodiments of the present disclosure is to provide a method for efficiently producing an unsaturated chlorofluorocarbon and composition.

### SOLUTION TO PROBLEM

In order to solve the above problem, the following embodiments are included.
[1] A method for producing a geometric isomer (isomer 2) represented by the following formula (1) by geometrically isomerizing a corresponding geometric isomer (isomer 1) represented by the following formula (1), the method including:
   contacting the geometric isomer (isomer 1) represented by the following formula (1) with a compound (A) which is at least one of a dichlorotrifluoropropane and hydrogen chloride in a gas phase,

      CF₃₋ₙHₙ-CX=CClH (1)
   (n is 0 or 1, and X is a fluorine atom or a hydrogen atom,
   when n is 0, X is a hydrogen atom and the dichlorotrifluoropropane is 1,1-dichloro-3,3,3-trifluoropropane, and
   when n is 1, X is a fluorine atom, and the dichlorotrifluoropropane is at least one of 1,1-dichloro-2,3,3-trifluoropropane and 1,2-dichloro-2,3,3-trifluoropropane).
[2] The method according to [1], in which the geometric isomer (isomer 1) represented by the formula (1) is a cis isomer, and the produced geometric isomer (isomer 2) is a trans isomer.
[3] The method according to [2], in which the geometric isomer (isomer 1) represented by the formula (1) is Z-1-chloro-3,3,3-trifluoropropene, and the produced geometric isomer (isomer 2) is E-1-chloro-3,3,3-trifluoropropene.
[4] The method according to [2], in which the geometric isomer (isomer 1) represented by the formula (1) is Z-1-chloro-2,3,3-trifluoropropene, and the produced geometric isomer (isomer 2) is E-1-chloro-2,3,3-trifluoropropene.
[5] The method according to any one of [2] to [4], in which the compound (A) is hydrogen chloride.
[6] The method according to [1], in which the geometric isomer (isomer 1) represented by the formula (1) is a trans isomer, and the produced geometric isomer (isomer 2) is a cis isomer.
[7] The method according to [6], in which the geometric isomer (isomer 1) represented by the formula (1) is E-1-chloro-3,3,3-trifluoropropene, and the produced geometric isomer (isomer 2) is Z-1-chloro-3,3,3-trifluoropropene.
[8] The method according to [6], in which the geometric isomer (isomer 1) represented by the formula (1) is E-1-chloro-2,3,3-trifluoropropene, and the produced geometric isomer (isomer 2) is Z-1-chloro-2,3,3-trifluoropropene.
[9] The method according to any one of [6] to [8], in which the compound (A) is a dichlorotrifluoropropane.
[10] The method according to any one of [1] to [9], in which the contacting is performed in the presence of at least one of a catalyst and a filler.
[11] The method according to any one of [1] to [10], in which the contacting is performed in the presence of activated carbon.
[12] The method according to any one of [1] to [11], in which the contacting is performed at a temperature higher than 150°C and lower than 500°C.
[13] A raw material composition for producing a geometric isomer (isomer 2) represented by the following formula (1) by geometrically isomerizing a corresponding geometric isomer (isomer 1) represented by the following formula (1), the raw material composition containing:
   the geometric isomer (isomer 1) represented by the following formula (1); and
   a compound (A) which is at least one of a dichlorotrifluoropropane and hydrogen chloride,

      CF₃₋ₙHₙ-CX=CClH (1)
   (n is 0 or 1, and X is a fluorine atom or a hydrogen atom,
   when n is 0, X is a hydrogen atom and the dichlorotrifluoropropane is 1,1-dichloro-3,3,3-trifluoropropane, and
   when n is 1, X is a fluorine atom, and the dichlorotrifluoropropane is at least one of 1,1-dichloro-2,3,3-trifluoropropane and 1,2-dichloro-2,3,3-trifluoropropane).
[14] A composition containing:
   a geometric isomer represented by the following formula (1); and
   a compound (A), in which the compound (A) is at least one of a dichlorotrifluoropropane and hydrogen chloride,

      CF₃₋ₙHₙ-CX=CClH (1)
   (n is 0 or 1, and X is a fluorine atom or a hydrogen atom,
   when n is 0, X is a hydrogen atom and the dichlorotrifluoropropane is 1,1-dichloro-3,3,3-trifluoropropane, and
   when n is 1, X is a fluorine atom, and the dichlorotrifluoropropane is at least one of 1,1-dichloro-2,3,3-trifluoropropane and 1,2-dichloro-2,3,3-trifluoropropane).

### ADVANTAGEOUS EFFECTS OF INVENTION

According to one embodiment of the present disclosure, it is possible to provide a method for efficiently producing an unsaturated chlorofluorocarbon, and composition.

### DESCRIPTION OF EMBODIMENTS

Embodiments of the present disclosure will be described below. However, the present disclosure can be implemented in various aspects without departing from the gist thereof, and should not be construed as being limited to the description of the embodiments illustrated below. In addition, even if there are other actions and effects different from the actions and effects brought about by the aspects of the following embodiments, it is understood that what is obvious from the description of the present description or what can be easily predicted by a person skilled in the art is brought about by the present disclosure.

In addition, in the present description, for halogenated hydrocarbons, the abbreviation of the compound is written in parentheses after the compound name, and the abbreviation is used in place of the compound name as necessary. In addition, for compounds that have a double bond in the molecule and have a trans isomer (E isomer) and a cis isomer (Z isomer) as isomers, the E isomer and Z isomer are indicated by (E) and (Z) at the end of the compound abbreviations, respectively. Abbreviations of compound names without (E) or (Z) at the end indicate E isomer and/or Z isomer.

In the present description, when the isomer 1 is a Z isomer, the corresponding isomer 2 is an E isomer. When the isomer 1 is an E isomer, the corresponding isomer 2 is a Z isomer.

The cis isomer of the isomer 1 is represented by the following structure.

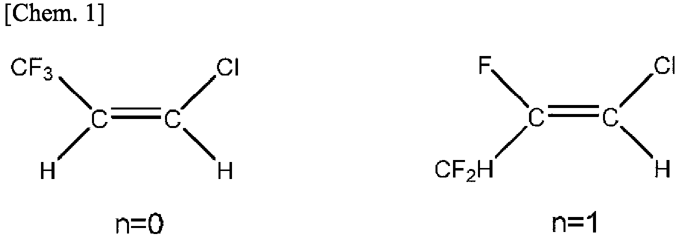

The trans isomer of the isomer 1 is represented by the following structure.

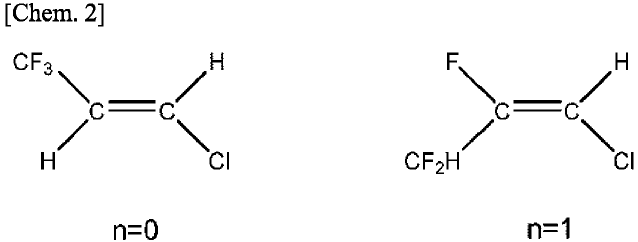

The isomer 1 is Z-1-chloro-3,3,3-trifluoropropene (cis isomer). E-1-chloro-3,3,3-trifluoropropene (trans isomer), Z-1-chloro-2,3,3-trifluoropropene (cis isomer), or E-1-chloro-2,3,3-trifluoropropene (trans isomer).

When the isomer 1 is Z-1-chloro-3,3,3-trifluoropropene, the corresponding isomer 2 is E-1 -chloro-3,3,3 -trifluoropropene.

When the isomer 1 is E-1-chloro-3,3,3-trifluoropropene, the corresponding isomer 2 is Z-1 -chloro-3,3,3 -trifluoropropene.

When the isomer 1 is Z-1-chloro-2,3,3-trifluoropropene, the corresponding isomer 2 is E-1 -chloro-2,3,3 -trifluoropropene.

When the isomer 1 is E-1-chloro-2,3,3-trifluoropropene, the corresponding isomer 2 is Z-1-chloro-2,3,3-trifluoropropene.

### [Overview of Production Method]

Hereinafter, a method (hereinafter also referred to as "the present production method") for producing 1-chloro-3,3,3-trifluoropropene (hereinafter also referred to as "1233zd") and 1-chloro-2,3,3-trifluoropropene (hereinafter also referred to as "1233yd") according to the present embodiment will be described.

The present production method is a method for producing a geometric isomer (isomer 2) represented by the following formula (1) by geometrically isomerizing a corresponding geometric isomer (isomer 1) represented by the following formula (1), the method including:
contacting the geometric isomer (isomer 1) represented by the following formula (1) with a compound (A), which is at least one of a dichlorotrifluoropropane and hydrogen chloride, as a accelerator in a gas phase.

   CF₃₋ₙHₙ-CX=CClH (1)
(n is 0 or 1, and X is a fluorine atom or a hydrogen atom,
when n is 0, X is a hydrogen atom and the dichlorotrifluoropropane is 1,1-dichloro-3,3,3-trifluoropropane, and
when n is 1, X is a fluorine atom, and the dichlorotrifluoropropane is at least one of 1,1-dichloro-2,3,3-trifluoropropane and 1,2-dichloro-2,3,3-trifluoropropane).

The compound (A) in the present production method is at least one of a dichlorotrifluoropropane and hydrogen chloride. The above compound (A) functions as an accelerator for the isomerization reaction.

When the isomer 1 is a cis isomer, the above compound (A) is preferably hydrogen chloride, but is not limited to this. For example, in an isomerization reaction for Z-1-chloro-3,3,3-trifluoropropene, hydrogen chloride is preferred as the accelerator, but a dichlorotrifluoropropane also functions as the accelerator to accelerate the isomerization reaction.

When the isomer 1 is a trans isomer the above compound (A) is preferably a dichlorotrifluoropropane, but is not limited to this. For example, in an isomerization reaction for E-1-chloro-3,3,3-trifluoropropene, the dichlorotrifluoropropane is preferred as the accelerator, but hydrogen chloride also functions as the accelerator to accelerate the isomerization reaction.

In the isomerization, the total amount of the compound (A) is preferably 0.001 mol or more to 1 mol or less, more preferably 0.005 mol or more to 0.5 mol or less, and still more preferably 0.01 mol or more to 0.2 mol or less, per 1 mol of the geometric isomer (isomer 1) represented by the formula (1), but is not limited to this.

The isomerization is performed in a gas phase. A batch system or a flow system can be applied to the isomerization, and a gas phase flow system with high industrial productivity is preferred.

The isomerization may be performed in the presence of at least one of a catalyst and a filler. Specifically, a reaction tube is filled with at least one of a catalyst and a filler, and the gaseous geometric isomer (isomer 1) represented by the formula (1) is brought into contact with the compound (A). The isomerization is preferably performed in the presence of a filler, but is not limited to this.

The isomerization reaction in the present embodiment is performed in the presence of the compound (A). Accordingly, the geometric isomerization from the geometric isomer (isomer 1) represented by the above formula (1) to the corresponding geometric isomer (isomer 2) can be efficiently performed. That is, the compound (A) functions as an accelerator for geometric isomerization of 1233zd or 1233yd.

When 1233zd is isomerized to obtain the corresponding geometric isomer, the dichlorotrifluoropropane is 1,1-dichloro-3,3,3-trifluoropropane (hereinafter also referred to as "243fa"). In the isomerization, since 243fa itself can also be converted to 1233zd, it contributes to the improvement of the production efficiency of 1233zd.

When 1233yd is isomerized to obtain the corresponding geometric isomer, the dichlorotrifluoropropane is at least one of 1,1-dichloro-2,3,3-trifluoropropane (hereinafter also referred to as "243eb") and 1,2-dichloro-2,3,3-trifluoropropane (hereinafter also referred to as "243ba"). In the isomerization, since 243eb or 243ba itself can also be converted to 1233yd, it contributes to the improvement of the production efficiency of 1233yd.

1233zd (Z) and 1233zd (E) can be separated by precision distillation due to the difference in boiling points. Therefore, when 1233zd (E) is isomerized to obtain 1233zd (Z), it is rational and preferred from the viewpoint of efficient use of raw materials that a product containing 1233zd (Z) obtained by isomerization is collected, 1233zd (Z) and 1233zd (E) are separated by distillation or the like, and then the recovered 1233zd (E) is used again as a raw material for isomerization.

Similarly, when 1233zd (Z) is isomerized to obtain 1233zd (E), it is rational and preferred from the viewpoint of efficient use of raw materials that a product containing 1233zd (E) obtained by isomerization is collected, 1233zd (E) and 1233zd (Z) are separated by distillation or the like, and then the recovered 1233zd (Z) is used again as a raw material for isomerization.

When isomerizing 1233zd (Z) as a raw material to 1233zd (E), the target 1233zd (E) may be mixed in the raw material 1233zd (Z), and it is preferable to use a raw material having a mass ratio of 1233zd (E) to 1233zd (Z), i.e., 1233zd (E)/1233zd (Z) = 1/99 or less in order to efficiently proceed the isomerization, 0.1/99.9 or less is more preferred, and 0.01/99.99 or less is still more preferred.

When isomerizing 1233zd (E) as a raw material to 1233zd (Z), the target 1233zd (Z) may be mixed in the raw material 1233zd (E), and it is preferable to use a raw material having a mass ratio of 1233zd (E) to 1233zd (Z), i.e., 1233zd (E)/1233zd (Z) = 10/1 or more in order to efficiently proceed the isomerization, 50/1 or more is more preferred, and 100/1 or more is still more preferred.

1233yd (Z) and 1233yd (E) can be separated by precision distillation due to the difference in boiling points. Therefore, when 1233yd (E) is isomerized to obtain 1233yd (Z), it is rational and preferred from the viewpoint of efficient use of raw materials that a product containing 1233yd (Z) obtained by isomerization is collected, 1233yd (Z) and 1233yd (E) are separated by distillation or the like, and then the recovered 1233yd (E) is used again as a raw material for isomerization.

Similarly, when 1233yd (Z) is isomerized to obtain 1233yd (E), it is rational and preferred from the viewpoint of efficient use of raw materials that a product containing 1233yd (E) obtained by isomerization is collected, 1233yd (E) and 1233yd (Z) are separated by distillation or the like, and then the recovered 1233yd (Z) is used again as a raw material for isomerization.

The compound (A) may be supplied to the reaction tube as a raw material together with the compound represented by the above formula (1) in the isomerization of the compound represented by the above formula (1), or may be supplied to a reaction tube separately from the raw material containing the compound represented by the above formula (1).

Examples of the catalyst that can be used in the isomerization include a metal catalyst. The metal catalyst is not particularly limited, and preferably contains at least one metal selected from the group consisting of aluminum, chromium, titanium, manganese, iron, nickel, cobalt, copper, magnesium, zirconium, molybdenum, zinc, tin, lanthanum, and antimony.

The metal catalyst may be a supported catalyst supported on a carrier such as activated carbon. Examples of the supported metal include, but not limited to, aluminum, chromium, titanium, manganese, iron, nickel, cobalt, copper, magnesium, zirconium, molybdenum, zinc, tin, lanthanum, and antimony. These metals are supported as fluorides, chlorides, fluorochlorides, oxyfluorides, oxychlorides, oxyfluorochlorides, and the like, and two or more metal compounds may be supported together.

As the carrier, not only activated carbon but also metals such as alumina, chromia, zirconia and titania can be used. From the viewpoint of reaction efficiency, it is preferable to use activated carbon as a carrier.

Examples of the filler can be used in the isomerization include, but not limited to, activated carbon, a stainless steel-made Raschig ring, a stainless steel-made net, a quartz-made Raschig ring, and a glass-made Raschig ring. From the viewpoint of reaction efficiency, it is preferable to use activated carbon.

Examples of the activated carbon include plant-based activated carbon made from charcoal, coconut shell coal, palm nuclear coal, and untreated ash, coal-based activated carbon made from peat, lignite, brown coal, bituminous coal, and anthracite, petroleum-based activated carbon made from petroleum residue and oil carbon, or synthetic resin-based activated carbon made from carbonized polyvinylidene chloride. The activated carbon used in the present embodiment can be selected from the following commercially available activated carbon. For example, coconut shell charcoal for gas purification and catalyst carrier (Granular SHIRASAGI GX, SX, CX, and XRC manufactured by Osaka Gas Chemicals Co., Ltd., PCB manufactured by Toyo Calgon, YASHIKORU manufactured by Taihei Chemical Industrial Co., Ltd., and Kuraray Coal GG, and GC) and the like are suitably used. In the present embodiment, when activated carbon is used as the filler, it is preferable to use activated carbon that does not support a metal. Activated carbon that does not support a metal is suitable from the viewpoint of cost and the viewpoint of waste disposal. In the present embodiment, the activated carbon that does not support a metal refers to activated carbon in which the metal content in the activated carbon catalyst is 0 mass% or more and 5 mass% or less, preferably 0 mass% or more and 1 mass% or less, and more preferably 0 mass% or more and 0.1 mass% or less.

The activated carbon used may be granular and may have spherical, fibrous, powdery or honeycomb morphology, as long as it is compatible with the reactor. The specific surface area and pore volume of the activated carbon are sufficient within the ranges of commercial product specifications, and the specific surface area is desirably greater than 400 m²/g, and more preferably 800 m²/g or more and 3000 m²/g or less. The pore volume is preferably greater than 0.1 cm³/g, and more preferably 0.2 cm³/g or more and 1.0 cm³/g or less.

The reaction temperature in the isomerization reaction is not particularly limited as long as it is equal to or higher than the boiling point of the isomer 1, and it is preferably higher than 150°C and lower than 500°C. When the reaction temperature in the isomerization reaction is higher than 150°C, the target product can be obtained in a good yield, and when it is lower than 500°C, impurities are hardly produced as a by-product, and a high-purity target product can be obtained. The reaction temperature in the isomerization reaction is preferably 180°C or higher and 380°C or lower, particularly preferably 220°C or higher and 330°C or lower, and more preferably higher than 250°C and 330°C or lower. The method of heating the reaction tube is not particularly limited, and examples thereof include a method of direct heating with an electric heater or burner, and a method of indirect heating using a molten salt or sand.

The reaction time in the isomerization reaction is defined by the "contact time" described below. That is, the volume of the reaction tube (the total volume of the catalyst and filler in the case of being used in the reaction tube) is set to A, and the volume of the raw material gas introduced into the reaction tube per second is set to B. B is calculated from the number of moles of the raw material and the diluent gas introduced per second, the pressure, and the temperature, assuming that the raw material gas is an ideal gas. At this time, the value obtained by dividing A by B (=A/B) is the contact time.

Since the contact time depends on the temperature (reaction temperature) and the shape of the reaction tube, and the catalyst, it is desirable to optimize the supply rate for the raw material by appropriately adjusting the supply rate for each of the set temperature, the shape of the reaction tube, and the catalyst type. From the viewpoint of recovering and reusing unreacted raw materials, it is preferable to employ a contact time that provides a raw material conversion rate of 5% or more, and more preferably, the contact time is optimized such that a conversion rate of 10% or more is obtained.

The contact time is not particularly limited, and is usually 10 seconds or longer and 180 seconds or shorter, and preferably 30 seconds or longer and 120 seconds or shorter.

As a preferred embodiment, when the reaction temperature is in the range of higher than 150°C and lower than 500°C, the contact time may be 10 seconds or longer and 180 seconds or shorter, but is not limited thereto.

The reaction pressure is not particularly limited, and it is preferable to perform the reaction at around normal pressure. A pressurized reaction of 1 MPa or more is not preferred since not only does it require an expensive pressure-resistant device, but there is also concern about the polymerization of the raw material or the product. In addition, the reaction can be performed using an inert gas such as nitrogen or argon as a diluent gas.

A reaction device that can be used for the isomerization preferably includes a reaction tube or a unit for introducing and discharging various gases. They are formed from materials that are highly resistant to hydrogen chloride. Such materials may be, for example, quartz, carbon, ceramics, stainless steel materials such as austenitic stainless steels, high nickel alloys such as Monel (registered trademark), Hastelloy (registered trademark), and Inconel (registered trademark), and copper clad steels, but are not limited thereto. The shape of the reaction tube is not particularly limited. The inside of the reaction tube may be empty, or the reaction tube may be provided with a filling object such as a static mixer, a Raschig ring, a Pall ring, or a wire mesh, which is inert to the reaction.

### [Overview of Composition]

The present disclosure also relates to the following composition.

A raw material composition for producing a geometric isomer (isomer 2) represented by the following formula (1) by geometrically isomerizing a corresponding geometric isomer (isomer 1) represented by the following formula (1), the composition containing:
the geometric isomer (isomer 1) represented by the following formula (1); and
a compound (A) which is at least one of a dichlorotrifluoropropane and hydrogen chloride,

   CF₃₋ₙHₙ-CX=CClH (1)
(n is 0 or 1, and X is a fluorine atom or a hydrogen atom,
when n is 0, X is a hydrogen atom and the dichlorotrifluoropropane is 1,1-dichloro-3,3,3-trifluoropropane, and
when n is 1, X is a fluorine atom, and the dichlorotrifluoropropane is at least one of 1,1-dichloro-2,3,3-trifluoropropane and 1,2-dichloro-2,3,3-trifluoropropane).

In addition, the present disclosure also relates to the following composition.

A composition containing:
a geometric isomer represented by the following formula (1); and
a compound (A), in which the compound (A) is at least one of a dichlorotrifluoropropane and hydrogen chloride,

   CF₃₋ₙHₙ-CX=CClH (1)
(n is 0 or 1, and X is a fluorine atom or a hydrogen atom,
when n is 0, X is a hydrogen atom and the dichlorotrifluoropropane is 1,1-dichloro-3,3,3-trifluoropropane, and
when n is 1, X is a fluorine atom, and the dichlorotrifluoropropane is at least one of 1,1-dichloro-2,3,3-trifluoropropane and 1,2-dichloro-2,3,3-trifluoropropane).

Each component in the above composition is as described above.

### [Examples]

Hereinafter, the production of the unsaturated chlorofluorocarbon according to the present disclosure will be specifically described with reference to Examples, but the present disclosure is not limited to these Examples.

Here, the composition analysis value "GC%" of the raw material and the reaction product represents the "GC area%" of the composition obtained by measuring the raw material and the reaction product by gas chromatography (detector: FID). Numbers below the number of displayed digits are rounded off. For example, 0.0 GC% indicates less than 0.05 GC%.

### 1. Geometric Isomerization of 1233zd (E)

### [Example 1]

A gas phase reaction device including a reaction tube filled with 100 cc of activated carbon (SHIRASAGI G2X manufactured by Osaka Gas Chemicals Co., Ltd.) was equipped with a metal electric heater and an external heating device (a mantle heater manufactured by TOKYO KIKI co. INC.), and the reaction device was heated while flowing a nitrogen gas therethrough at a flow rate of about 50 mL/min.

Next, while flowing a nitrogen gas at a flow rate of 50 mL/min, a mixed liquid containing 1233zd (E) (85.8 GC%), 1233zd (Z) (0.0 GC%), 243fa (13.6 GC%), and other components (0.6 GC%) as the starting material was supplied to the reaction tube through a vaporizer at a flow rate of 0.6 g/min. When the flow rate of the raw material was stabilized, the introduction of the nitrogen gas was stopped, and the temperature inside the reaction tube during this time was 250°C. After confirming that the reaction had stabilized, the gas discharged from the reactor was blown into water to remove the acid gas, and then the product was analyzed by gas chromatography. Table 1 shows the reaction temperature, the contact time, and the gas chromatography analysis result. In addition, 1233zd E/Z indicates a generation ratio of 1233zd (E) to 1233zd (Z) (that is, the value obtained by dividing the generation amount (GC%) of 1233zd (E) by the generation amount (GC%) of 1233zd (Z)).

Table 1 also shows the composition of the above raw material.

### [Examples 2 to 5]

The isomerization reaction was performed in the same manner as in Example 1, except that the reaction temperature and the contact time were changed. Table 1 shows the reaction temperature, the contact time, and the gas chromatography analysis result of the products in the isomerization reaction in Examples 2 to 5.

**[Table 1]**

| | Reaction temperature | Contact time | Composition of reaction product (GC%) | | | | 1233zd E/Z |
|---|---|---|---|---|---|---|---|
| | °C | s | 1233zd (E) | 1233zd (Z) | 243fa | Others | |
| | Raw material | | 85.8 | 0.0 | 13.6 | 0.6 | |
| Example 1 | 250 | 60 | 96.8 | 2.7 | 0.1 | 0.4 | 35.9 |
| Example 2 | 250 | 120 | 93.0 | 6.2 | 0.1 | 0.7 | 15.0 |
| Example 3 | 300 | 60 | 88.6 | 10.6 | 0.0 | 0.8 | 8.4 |
| Example 4 | 300 | 120 | 86.4 | 12.3 | 0.0 | 1.3 | 7.0 |
| Example 5 | 350 | 60 | 83.4 | 13.3 | 0.0 | 3.3 | 6.3 |

As the raw material in Examples 1 to 5, a mixture of 1233zd (E):243fa = 6.9:1 in terms of molar ratio (i.e., the amount of 243fa to 1 mol of 1233zd (E) was 0.145 mol) was used. As a result of gas chromatography analysis on this raw material, 1233zd (E) was 85.8 GC%, 1233zd (Z) was 0.0 GC%, 243fa was 13.6 GC%, and other components were 0.6 GC%.

In order to confirm the conversion of 243fa to 1233zd (E) or 1233zd (Z), a gas phase dehydrochlorination was performed on 243fa.

### [Reference Example 1]

While flowing a nitrogen gas at a flow rate of 50 mL/min, a mixed liquid containing 243fa (95.2 GC%) and 1233zd (Z) (0.0 GC%) as the starting material for the isomerization reaction was vaporized through a vaporizer and supplied to a reaction tube at a flow rate of 1.5 g/min. When the flow rate of the raw material was stabilized, the introduction of the nitrogen gas was stopped, and the temperature inside the reaction tube during this time was 250°C. After confirming that the reaction had stabilized, the gas discharged from the reactor was blown into water to remove the acid gas, and then the product was analyzed by gas chromatography. Table 2 shows the reaction temperature, the contact time, and the gas chromatography analysis result.

Table 2 also shows the composition of the above raw material.

### [Reference Examples 2 to 4]

The isomerization reaction was performed in the same manner as in Reference Example 1, except that the reaction temperature and the contact time were changed. Table 2 shows the reaction temperature, the contact time, and the gas chromatography analysis result of the products in the isomerization reaction in Reference Examples 2 to 4.

**[Table 2]**

| | Reaction temperature | Contact time | Composition of reaction product (GC%) | | | | 1233zd E/Z |
|---|---|---|---|---|---|---|---|
| | °C | s | 1233zd (E) | 1233zd (Z) | 243fa | Others | |
| | Raw material | | 0.0 | 0.0 | 95.2 | 4.8 | |
| Reference Example 1 | 250 | 30 | 60.7 | 9.1 | 26.1 | 4.1 | 6.7 |
| Reference Example 2 | 250 | 60 | 79.3 | 11.6 | 5.3 | 3.8 | 6.8 |
| Reference Example 3 | 250 | 120 | 83.1 | 12.3 | 0.6 | 4.0 | 6.8 |
| Reference Example 4 | 300 | 30 | 83.9 | 12.0 | 0.4 | 3.7 | 7.0 |

As is clear from Table 2, 243fa is converted to 1233zd, and the product composition is such that 1233zd (E) is the main product and 1233zd (Z) is the main by-product. In addition, it can be seen that when the reaction temperature is raised, the conversion rate of 243fa is improved, but the generation ratio of 1233zd (E) to 1233zd (Z) does not change. The generation ratio of 1233zd (E) to 1233zd (Z) is about 7 (1233zd E/Z = about 7) regardless of the reaction temperature and the contact time.

In order to confirm the reaction accelerating effect of 243fa, Table 3 shows theoretical amounts of 1233zd (Z) and 1233zd (E) when all of the added 243fa is converted to 1233zd (Z) or 1233zd (E) using Example 3 in Table 1.

**[Table 3]**

| | Reaction temperature | Contact time | Composition of reaction product (GC%) | | | |
|---|---|---|---|---|---|---|
| | °C | s | 1233zd (E) | 1233zd (Z) | 243fa | Others |
| | Raw material | | 85.8 | 0.0 | 13.6 | 0.6 |
| Example 3 | 300 | 60 | 88.6 | 10.6 | 0.0 | 0.8 |
| Theoretical amount when all added 243fa is converted | | | 11.9 | 1.7 | | |

As is clear from Table 3, in Example 3, 1233zd (Z) is generated in an amount equal to or more than the theoretical amount of 1233zd (Z) generated from 243fa, and it is considered that the effect of 243fa acting as an accelerator in the isomerization reaction contributes more than the effect of converting 243fa to 1233zd (Z).

### [Comparative Example 1]

A gas phase reaction device including a reaction tube filled with 100 cc of activated carbon (SHIRASAGI G2X manufactured by Osaka Gas Chemicals Co., Ltd.) was equipped with a metal electric heater and an external heating device (a mantle heater manufactured by TOKYO KIKI co. INC.), and the reaction device was heated while flowing a nitrogen gas therethrough at a flow rate of about 50 mL/min.

Next, while flowing a nitrogen gas at a flow rate of 50 ml/min, a mixed liquid containing 1233zd (E) (> 99.9 GC%) and 1233zd (Z) (0.0 GC%) as the starting material was supplied to the reaction tube through a vaporizer at a flow rate of 0.6 g/min. When the flow rate of the raw material was stabilized, the introduction of the nitrogen gas was stopped, and the temperature inside the reaction tube during this time was 300°C. After confirming that the reaction had stabilized, the gas discharged from the reactor was blown into water to remove the acid gas, and then the product was analyzed by gas chromatography. Table 4 shows the reaction temperature, the contact time, and the gas chromatography analysis result.

Table 4 also shows the composition of the above raw material.

### [Comparative Example 2]

The isomerization reaction was performed in the same manner as in Comparative Example 1, except that the contact time was changed. Table 4 shows the reaction temperature, the contact time, and the gas chromatography analysis result of the product in the isomerization reaction in Comparative Example 2.

**[Table 4]**

| | Reaction temperature | Contact time | Composition of reaction product (GC%) | | | | 1233zd E/Z |
|---|---|---|---|---|---|---|---|
| | °C | s | 1233zd (E) | 1233zd (Z) | 243fa | Others | |
| | Raw material | | > 99.9 | 0.0 | 0.0 | 0.0 | |
| Comparative Example 1 | 300 | 60 | 97.1 | 2.8 | 0.0 | 0.1 | 34.7 |
| Comparative Example 2 | 300 | 120 | 93.5 | 6.3 | 0.0 | 0.2 | 14.8 |

As is clear from a comparison between Table 1 and Table 4, it can be seen that the isomerization of 1233zd (E) in the presence of 243fa improves the conversion rate from 1233zd (E) to 1233zd (Z) at each stage. Particularly when the reaction temperature is 300°C, the conversion rate is significantly improved.

### 2. Geometric Isomerization of 1233zd (Z)

### [Example 6]

The same operation as in Example 1 was performed, except that a mixed liquid containing 1233zd (E) (0.0 GC%) and 1233zd (Z) (> 99.9 GC%) was used as the starting material for the isomerization reaction, and hydrogen chloride (HCl) as the compound (A) was supplied to the reaction tube at a supply rate of 5 ml/min. The amount of HCl was 0.05 mol per 1 mol of 1233zd (Z) in the starting material. Table 5 shows the gas chromatography analysis result.

### [Examples 7 to 10]

The isomerization reaction was performed in the same manner as in Example 6, except that the reaction temperature and the amount of HCl added were changed. Table 5 shows the reaction temperature, the contact time, the amount of HCl added, and the gas chromatography analysis result of the products in the isomerization reaction in Examples 7 to 10.

**[Table 5]**

| | Reaction temperature | Contact time | HCl | Composition of reaction product (GC%) | | | | 1233zd E/Z |
|---|---|---|---|---|---|---|---|---|
| | °C | s | mol % | 1233zd (E) | 1233zd (Z) | 243fa | Others | |
| | Raw material | | | 0.0 | > 99.9 | 0.0 | 0.0 | |
| Example 6 | 250 | 60 | 5 | 15.4 | 83.9 | 0.1 | 0.6 | 0.2 |
| Example 7 | 250 | 60 | 10 | 29.7 | 68.9 | 0.4 | 1.0 | 0.4 |
| Example 8 | 300 | 60 | 5 | 50.6 | 48.8 | 0.0 | 0.6 | 1.0 |
| Example 9 | 300 | 60 | 10 | 80.3 | 18.1 | 0.0 | 1.7 | 4.4 |
| Example 10 | 350 | 60 | 5 | 83.7 | 13.8 | 0.0 | 2.6 | 6.1 |

### [Comparative Example 3]

A gas phase reaction device including a reaction tube filled with 100 cc of activated carbon (SHIRASAGI G2X manufactured by Osaka Gas Chemicals Co., Ltd.) was equipped with a metal electric heater and an external heating device (a mantle heater manufactured by TOKYO KIKI co. INC.), and the reaction device was heated while flowing a nitrogen gas therethrough at a flow rate of about 50 mL/min.

Next, while feeding nitrogen at 50 ml/min, a mixed liquid containing 1233zd (E) (0.0 GC%) and 1223zd (Z) (> 99.9 GC%) as the starting material was supplied to the reaction tube through a vaporizer at a flow rate of 0.6 g/min. When the flow rate of the raw material was stabilized, the introduction of the nitrogen gas was stopped, and the temperature inside the reaction tube during this time was 250°C. After confirming that the reaction had stabilized, the gas discharged from the reactor was blown into water to remove the acid gas, and then the product was analyzed by gas chromatography. Table 6 shows the gas chromatography analysis result.

### [Comparative Examples 4 and 5]

The isomerization reaction was performed in the same manner as in Comparative Example 3, except that the reaction temperature was changed. Table 6 shows the reaction temperature, the contact time, and the gas chromatography analysis result of the products in the isomerization reaction in Comparative Examples 4 and 5.

**[Table 6]**

| | Reaction temperature | Contact time | Composition of reaction product (GC%) | | | | 1233zd E/Z |
|---|---|---|---|---|---|---|---|
| | °C | s | 1233zd (E) | 1233zd (Z) | 243fa | Others | |
| | Raw material | | 0.0 | > 99.9 | 0.0 | 0.0 | |
| Comparative Example 3 | 250 | 60 | 3.8 | 95.7 | 0.0 | 0.5 | 0.0 |
| Comparative Example 4 | 300 | 60 | 28.8 | 69.9 | 0.0 | 1.3 | 0.4 |
| Comparative Example 5 | 350 | 60 | 82 | 15.8 | 0.0 | 2.2 | 5.2 |

As is clear from Table 5 and Table 6, it can be seen that the isomerization of 1233zd (Z) in the presence of HCl particularly improves the conversion rate from 1233zd (Z) to 1233zd (E). In addition, it is also seen that increasing the amount of HCl added increases the accelerating effect. This result suggests the progress of isomerization via 243fa due to the addition and elimination of HCl. In addition, in the isomerization of 1233zd (E), the reason why 243fa accelerates the isomerization is presumed to be that hydrogen chloride generated by the decomposition of these accelerators contributes to the reaction.

In addition, as is clear from Table 4 and Table 6, it can be seen that the conversion from 1233zd (Z) to 1233zd (E) can be performed similar to the conversion from 1233zd (E) to 1233zd (Z). Therefore, it can be presumed that the isomerization of 1233zd (Z) in the presence of 243fa can particularly improve the conversion rate from 1233zd (Z) to 1233zd (E).

### 3. Geometric Isomerization of 1233yd

The isomerization reaction is performed in the same method as in Examples 1 to 5 except that a starting material containing at least one of 1233yd (E) and 1233yd (Z) and at least one of 243eb and 243ba is used. In addition, the isomerization reaction from 1233yd (E) to 1233yd (Z) or the isomerization reaction from 1233yd (Z) to 1233yd (E) is performed in the same method as in Examples 6 to 10, except that a liquid containing at least one of 1233yd (E) and 1233yd (Z) is used instead of the mixed liquid containing 1233zd (E) (0.0 GC%) and 1233zd (Z) (> 99.9 GC%). The products are analyzed by gas chromatography.

In the isomerization reaction from 1233yd (E) to 1233yd (Z) or the isomerization reaction from 1233yd (Z) to 1233yd (E), by containing at least one of 243eb, 243ba and HCl as the compound (A) in the raw material, the isomerization reaction from 1233yd (E) to 1233yd (Z) or the conversion rate from 1233yd (Z) to 1233yd (E) can also be improved particularly. It is presumed that when using 243eb or 243ba, 1233yd and hydrogen chloride are generated by decomposition of 243eb or 243ba, and this hydrogen chloride contributes to the reaction.

### INDUSTRIAL APPLICABILITY

According to one embodiment of the present disclosure, it is possible to provide a method for efficiently producing an unsaturated chlorofluorocarbon, and composition.

Although the present disclosure has been described in detail and with reference to specific examples, it will be apparent to those skilled in the art that various changes and modifications can be made without departing from the spirit and scope of the present disclosure.

The present application is based on a Japanese Patent Application (No. 2020-212438) filed on December 22, 2020, the contents of which are incorporated herein by reference.

## Claims

1. A method for producing a geometric isomer (isomer 2) represented by the following formula (1) by geometrically isomerizing a corresponding geometric isomer (isomer 1) represented by the following formula (1), the method comprising:
contacting the geometric isomer (isomer 1) represented by the following formula (1) with a compound (A) which is at least one of a dichlorotrifluoropropane and hydrogen chloride in a gas phase,
CF₃₋ₙHₙ-CX=CClH (1)
wherein, n is 0 or 1, and X is a fluorine atom or a hydrogen atom,
when n is 0, X is a hydrogen atom and the dichlorotrifluoropropane is 1,1-dichloro-3,3,3-trifluoropropane, and
when n is 1, X is a fluorine atom, and the dichlorotrifluoropropane is at least one of 1,1-dichloro-2,3,3-trifluoropropane and 1,2-dichloro-2,3,3-trifluoropropane.

2. The method according to claim 1, wherein the geometric isomer (isomer 1) represented by the formula (1) is a cis isomer, and the produced geometric isomer (isomer 2) is a trans isomer.

3. The method according to claim 2, wherein the geometric isomer (isomer 1) represented by the formula (1) is Z-1-chloro-3,3,3-trifluoropropene, and the produced geometric isomer (isomer 2) is E-1-chloro-3,3,3-trifluoropropene.

4. The method according to claim 2, wherein the geometric isomer (isomer 1) represented by the formula (1) is Z-1-chloro-2,3,3-trifluoropropene, and the produced geometric isomer (isomer 2) is E-1-chloro-2,3,3-trifluoropropene.

5. The method according to any one of claims 2 to 4, wherein the compound (A) is hydrogen chloride.

6. The method according to claim 1, wherein the geometric isomer (isomer 1) represented by the formula (1) is a trans isomer, and the produced geometric isomer (isomer 2) is a cis isomer.

7. The method according to claim 6, wherein the geometric isomer (isomer 1) represented by the formula (1) is E-1-chloro-3,3,3-trifluoropropene, and the produced geometric isomer (isomer 2) is Z-1-chloro-3,3,3-trifluoropropene.

8. The method according to claim 6, wherein the geometric isomer (isomer 1) represented by the formula (1) is E-1-chloro-2,3,3-trifluoropropene, and the produced geometric isomer (isomer 2) is Z-1-chloro-2,3,3-trifluoropropene.

9. The method according to any one of claims 6 to 8, wherein the compound (A) is a dichlorotrifluoropropane.

10. The method according to any one of claims 1 to 9, wherein the contacting is performed in the presence of at least one of a catalyst and a filler.

11. The method according to any one of claims 1 to 10, wherein the contacting is performed in the presence of activated carbon.

12. The method according to any one of claims 1 to 11, wherein the contacting is performed at a temperature higher than 150°C and lower than 500°C.

13. A raw material composition for producing a geometric isomer (isomer 2) represented by the following formula (1) by geometrically isomerizing a corresponding geometric isomer (isomer 1) represented by the following formula (1), the raw material composition comprising:
the geometric isomer (isomer 1) represented by the following formula (1); and
a compound (A) which is at least one of a dichlorotrifluoropropane and hydrogen chloride,
CF₃₋ₙHₙ-CX=CClH (1)
wherein, n is 0 or 1, and X is a fluorine atom or a hydrogen atom,
when n is 0, X is a hydrogen atom and the dichlorotrifluoropropane is 1,1-dichloro-3,3,3-trifluoropropane, and
when n is 1, X is a fluorine atom, and the dichlorotrifluoropropane is at least one of 1,1-dichloro-2,3,3-trifluoropropane and 1,2-dichloro-2,3,3-trifluoropropane.

14. A composition comprising:
a geometric isomer represented by the following formula (1); and
a compound (A), wherein
the compound (A) is at least one of a dichlorotrifluoropropane and hydrogen chloride,
CF₃₋ₙHₙ-CX=CClH (1)
wherein, n is 0 or 1, and X is a fluorine atom or a hydrogen atom,
when n is 0, X is a hydrogen atom and the dichlorotrifluoropropane is 1,1-dichloro-3,3,3-trifluoropropane, and
when n is 1, X is a fluorine atom, and the dichlorotrifluoropropane is at least one of 1,1-dichloro-2,3,3-trifluoropropane and 1,2-dichloro-2,3,3-trifluoropropane.
